# EUROPEAN PATENT SPECIFICATION

(11) **EP 3 406 239 B1**
(45) Date of publication and mention of the grant of the patent: **20.10.2021**
(21) Application number: 17172196.2
(22) Date of filing: 22.05.2017
(51) Int. Cl.: A61K 9/08, A61K 47/10, A61K 31/192

(54) **PALATABLE KETOPROFEN LYSINE SALT SOLUTIONS**
WOHLSCHMECKENDE KETOPROFEN-LYSIN-SALZ-LÖSUNGEN
SOLUTIONS DE SEL DE LYSINE DE KÉTOPROFÈNE AGRÉABLES AU GOÛT

(43) Date of publication of application: 28.11.2018
(73) Proprietor: Epifarma Srl, 85033 Episcopia (PZ) (IT)
(72) Inventor: IRIANNI, Guseppe, 85033 EPISCOPIA (PZ) (IT); MACCARI, Giuseppe, 29016 CORTEMAGGIORE (PC) (IT); AGOSTINO GIAMMILLARI, Salvatore, 29016 CORTEMAGGIORE (PC) (IT)
(74) Representative: Bianchetti & Minoja SRL

(56) References cited:
- EP-A1- 2 266 556
- WO-A1-95/07079
- WO-A1-99/52528
- WO-A1-2005/058276
- US-A1- 2004 116 528

## Description

This invention relates to ketoprofen lysine salt aqueous compositions which also comprise sorbitol, sodium saccharin, mint flavour, licorice flavour, methyl-4-hydroxybenzoate sodium, and having a pH comprised between 5.5 and 6.5.

These compositions are useful as over-the-counter analgesics and antipyretics.

### Background of the invention

Ketoprofen ((R,S)-2-(3-benzoylphenyl)-propionic acid) is a well-known non-steroidal anti-inflammatory drug (NSAID) It is currently sold worldwide as an over-the-counter NSAID analgesic and antipyretic in various forms, most notably in its lysine salt form.

When administered orally, ketoprofen is known to have a very unpleasant taste and strong throat burning sensation after swallowing, which makes compliance difficult particularly in children and the elderly. Solutions to this problem have been to administer the compound in the form of granules that have taste-masking properties.

There is however a need to provide ketoprofen lysine salt in solution as over-the-counter medications that can be ingested readily, which is palatable to the person ingesting it, and which is safe and stable over time.

### PRIOR ART

Passali et al. (Clin Ther. 2001, 23(9), 1508) disclose the use of a ketoprofen lysine salt mouthwash for treating oropharyngeal pain. Panerai et al. (Trends Med 2012; 12(4): 159-167) disclose orodispersible granules of ketoprofen lysine salt. Comoglu et al, (Pharm Dev Technol. 2016 Dec;21(8):901-908)) disclose solid, orally-disintegrating, ketoprofen formulations that include a taste-masking polymer.

WO99/52528 discloses pharmaceutical preparations containing ketoprofen salts with glucosamine, proline or hydroxyproline and particularly a mouthwash solution comprising ketoprofen glucosamine salt, sorbitol, saccharin, mint flavouring and water (Example 17).

### DETAILED DESCRIPTION OF THE INVENTION

We have found that when ketoprofen is formulated as an aqueous solution according to the claims with sorbitol, it becomes surprisingly palatable if added with a combination of the following taste-masking agents: sodium saccharin, mint flavour and licorice flavour. As it would otherwise fail to fulfil the safety test of Eur. Pharm. 5.1.3, such pharmaceutical solution must be added with an antimicrobial, such as methyl-4-hydroxybenzoate sodium, if safety requirements are to be met.

We have found that the addition of methyl-4-hydroxybenzoate sodium also improves the palatability of the composition.

Methyl-4-hydroxybenzoate sodium typically exhibits antimicrobial activity between pH 4 and 8 but its preservative efficacy decreases with increasing pH due to ionization in solution.

We have found that acceptable antimicrobial activities are obtained at a pH between 5.5 and 6.5. Most importantly, we found that when the pH is adjusted to 6, with monobasic sodium phosphate for example, the taste improves even better.

Accordingly, a pharmaceutical composition is provided in the form of an oral aqueous solution comprising ketoprofen lysine salt, sorbitol, sodium saccharin, mint flavour and licorice flavour and methyl-4-hydroxybenzoate sodium, wherein the pH of said composition is between 5.5 and 6.5, preferably pH 6.

In a specific embodiment, the pH is adjusted by addition with monobasic sodium phosphate.

In another embodiment, the composition is a ready-to-use composition.

We have also found that the compositions of the invention should be protected from UV radiations to prolong their shelf life. This can be achieved by packaging such compositions in a container made of either amber glass, amber polyethylene (PET) or non-translucent (opaque) PET.

The compositions of the invention are useful as medicaments, particularly to treat pain or fever.

The invention is now described by means of non-limiting examples.

### EXAMPLES

### Materials & Methods

### Example 1: Palatability tests of various compositions

Batches were submitted to a panel test of 4 people. Palatability was evaluated by each panelist and a score in a scale from "----" = "really bad mouth feeling, with typical throat burning sensation" to "++++" = "pleasant taste without feeling any throat burning sensation" was assigned to each one of the samples tested.

Licorice flavour 2742/AM, Mint flavour 2621/AZ and Mint/Licorice flavour 2742/V were purchased from Flavourland s.r.l.- Via D'Annunzio ang. Via Piave - 20016 PERO - MILANO - ITALY; and the codes 2742/AM, 2621/AZ and 2742/V are those of the manufacturer.

Results are shown in Table 1 below. Only samples comprising ketoprofen lysine salt, sorbitol, sodium saccharin, mint flavour, licorice flavour, methyl-4-hydroxybenzoate sodium, and having a pH comprised between 5.5 and 6.5 are according to the claims.

**Table 1**

| | | **COMPOSITION** % **w/V (g/L)** | | | | | | | |
|---|---|---|---|---|---|---|---|---|---|
| | | **A** | **B** | **C** | **D** | **E** | **F** | **G** | **H** |
| Ketoprofen lysine salt | | 0.572 | 0.572 | 0.572 | 0.572 | 0.572 | 0.572 | 0.572 | 0.572 |
| Liquid sorbitol 70% nc | | 40.000 | 40.000 | 40.000 | 40.000 | 40.000 | 45.000 | 45.000 | 45.000 |
| Sodium saccharin | | 0.070 | 0.070 | 0.070 | 0.070 | 0.070 | 0.070 | 0.070 | 0.070 |
| Licorice flavour 2742/AM | | 0 | 0 | 0 | 0 | 0.05 | 0.050 | 0 | 0.050 |
| Mint flavour 2621/AZ | | 0 | 0 | 0.1 | 0.2 | 0.15 | 0.150 | 0 | 0.150 |
| Mint/Licorice flavour 2742/V | | 0 | 0.1 | 0 | 0 | 0 | 0 | 0.200 | 0 |
| Na Methyl-p-hydroxy-benzoate | | 0 | 0 | 0 | 0 | 0 | 0.115 | 0.115 | 0.115 |
| Sodium phosphate monobasic pH = 6.0 | | 0 | 0 | 0 | 0 | 0 | 0 | 0 | q.s. pH=6.0 |
| Palatability, Panelist # | 1 | - | +/- | - | - | + | +++ | + | +++ |
| | 2 | -/+ | + | - | - | + | ++ | ++ | ++++ |
| | 3 | - | +/- | - | - | ++ | +++ | + | ++ |
| | 4 | - | + | - | - | + | +++ | ++ | ++++ |

### Example 2: The protection from UV radiation prolongs shelf life.

Composition H of example 1 was packaged in either an amber glass vial or a clear glass vial and subjected to stability analysis as shown in the tables 2 and 3 below:

**Table 2**

| ***IN AMBER GLASS VIAL*** | | | | | |
|---|---|---|---|---|---|
| ***Test*** | ***Specification*** | ***Initial time t₀*** | ***1 month 40°C*/*75% RH*** | ***1 month 60°C*** | ***ICH Photo-stability(primary packaging)*** |
| Appearance of solution | Clear, colourless solution | Complies | Complies | Yellow | Slightly white opaque |
| pH (as is) | Report result | 6.0 | 5.90 | 5.5 | 5.8 |
| Relative Density at 20°C | Report result | 1.110 | 1.110 | 1.110 | 1.110 |
| Assay - HPLC*: Ketoprofen Lysine salt | 90.0 - 110.0% of label claim | 99.7% | 95.1% | 95.2% | 90.2%** |

| Related Substances (HPLC) - (Reporting threshold ≥ 0.10%) | | | | | |
|---|---|---|---|---|---|
| Any unspecified degradation product | Report result | < 0.10% | < 0.10% | 0.15% (RRT 0.42) | 0.77%% (RRT 0.72) |
| Total degradation product | Report result | < 0.10% | < 0.10% | 0.15% | 1.12% |
| Assay - HPLC: Sodium Methyl-parahydroxybenzoate | 90.0 - 110.0% of label claim | 97.3% | 91.3% | 82.0% | 93.1% |

| | | | | | |
|---|---|---|---|---|---|
| *as free acid ** probably due to precipitation of API | | | | | |

**Table 3**

| ***IN CLEAR GLASS VIAL*** | | | | | |
|---|---|---|---|---|---|
| ***Test*** | ***Specification*** | ***Initial time*** | ***1 month 40°C*/*75% RH*** | ***1 month 60°C*** | ***ICH Photo-stability (primary packaging)*** |
| Appearance of solution | Clear, colourless solution | Complies | Complies | Cloudy yellow | Cloudy, white opaque |
| pH (as is) | Report result | 6.0 | 6.0 | 5.5 | 6.0 |
| Relative Density at 20°C | Report result | 1.110 | 1.110 | 1.110 | 1.110 |
| Assay - HPLC*: Ketoprofen Lysine salt | 90.0 - 110.0% of label claim | 98.4% | 95.2% | 94.4% | 24.5%** |

| Related Substances (HPLC) - (Reporting threshold ≥ 0.10%) | | | | | |
|---|---|---|---|---|---|
| Any unspecified degradation product | Report result | < 0.10% | < 0.10% | 0.35% (RRT 0.72) | 0.10% (RRT 0.24) |
| Total degradation product | Report result | < 0.10% | < 0.10% | 0.76% | 0.27% |
| Assay - HPLC: Sodium Methylparahydroxy-benzoate | 90.0 - 110.0% of label claim | 96.1% | 91.2% | 82.9% | 86.8% |

| | | | | | |
|---|---|---|---|---|---|
| *as free acid ** due to precipitation of API | | | | | |

### Example 3: pH 5.5 composition stability

| **Composition J** | **% w/V (g/L)** |
|---|---|
| Ketoprofen Lysine salt | 0.572 |
| Liquid sorbitol 70% nc | 45.000 |
| Sodium saccharine | 0.070 |
| Licorice flavour 2742/AM | 0.050 |
| Mint flavour 2621/AZ | 0.150 |
| Methyl-p-hydroxybenzoate sodium | 0.115 |
| Sodium phosphate monobasic dihydrate | q.s. pH=5.5 |
| Purified water | q.s. to 100 |

Composition J was packaged in either a PET amber vial or a PET white opaque vial and subjected to stability analysis as shown in the tables 4 and 5 below:

**Table 4**

| **Composition J in a PET white opaque vial** | | | | | | | | | | | |
|---|---|---|---|---|---|---|---|---|---|---|---|
| ***Test*** | ***Specification*** | ***Initial time*** | ***1 month 25°C*/*60 % RH*** | ***3 months 25°C*/*60 % RH*** | ***6 months 25°C*/*60* % *RH*** | ***1 month 40°C*/*75* % *RH*** | ***3 months 40°C*/*75* % *RH*** | ***6 months 40°C*/*75*% *RH*** | ***15 days 60°C*** | ***1 month 60°C*** | ***ICH Photo-stability (primary packaging)*** |
| Appearance of solution | Clear, colourless solution | Complies | Complies | Complies | Complies | Complies | Light yellow | Light yellow | Light yellow | Yellow | Complies |
| pH (as is) | Report result | 5.8 | TNP | 5.5 | 5.7 | TNP | 5.5 | 5.5 | TNP | 5.4 | 5.7 |
| Relative Density at 20°C | Report result | 1.122 | TNP | TNP | 1.120 | TNP | TNP | 1.120 | TNP | 1.122 | 1.122 |
| Assay - HPLC*: Ketoprofen Lysine salt | 90.0 - 110.0% of label claim | 99.5% | 103.2% | 100.4% | 99.0% | 101.8% | 100.7% | 100.1% | 99.4% | 99.1% | 100.0^% |

| Related Substances (HPLC) - (Reporting threshold ≥ 0.10%) | | | | | | | | | | | |
|---|---|---|---|---|---|---|---|---|---|---|---|
| Any unspecified degradation product | Report result | < 0.10% | < 0.10% | < 0.10% | < 0.10% | < 0.10% | < 0.10% | < 0.10% | < 0.10% | < 0.10% | 0.10% (Imp. A) |
| Total degradation product | Report result | < 0.10% | < 0.10% | < 0.10% | < 0.10% | < 0.10% | < 0.10% | < 0.10% | < 0.10% | < 0.10% | 0.10% |
| Assay HPLC: Sodium Methylparahydroxybenzoate | 90.0 - 110.0% of label claim | 92.0% | 93.2% | 91.3% | 87.6% | 90.0% | 86.6% | 83.0% | 79.8% | 67.0% | 99.2% |

| | | | | | | | | | | | |
|---|---|---|---|---|---|---|---|---|---|---|---|
| *as free acid; TNP = Test not performed | | | | | | | | | | | |

**Table 5**

| ***Composition J in a PET amber vial*** | | | | | | | |
|---|---|---|---|---|---|---|---|
| ***Test*** | ***Specification*** | ***Initial time*** | ***1 month 40°C*/*75% RH*** | ***3 months 40°C*/*75% RH*** | ***15 days 60°C*** | ***1 month 60°C*** | ***ICH Photo-stability (primary packaging)*** |
| Appearance of solution | Clear, colourless solution | Complies | Complies | Light yellow | Light yellow | Yellow | White cloudy |
| pH (as is) | Report result | 5.8 | TNP | 5.5 | TNP | 5.6 | 5.7 |
| Relative Density at 20°C | Report result | 1.122 | TNP | 1.122 | TNP | 1.122 | 1.122 |
| Assay - HPLC*: Ketoprofen Lysine salt | 90.0 - 110.0% of label claim | 99.5% | 98.4% | 101.1% | 99.8% | 99.8% | 86.3%** |

| Related Substances (HPLC) - (Reporting threshold ≥ 0.10%) | | | | | | | |
|---|---|---|---|---|---|---|---|
| Any unspecified degradation product | Report result | < 0.10% | < 0.10% | < 0.10% | < 0.10% | < 0.10% | 0.72% (Imp. A) |
| Total degradation product | Report result | < 0.10% | < 0.10% | < 0.10% | < 0.10% | < 0.10% | 0.92% |
| Assay - HPLC: Sodium Methylparahydro xybenzoate | 90.0 - 110.0% of label claim | 92.0% | 89.6% | 88.3% | 90.4% | 78.8% | 88.2% |

| | | | | | | | |
|---|---|---|---|---|---|---|---|
| *as free acid ** due to precipitation of API TNP = Test not performed | | | | | | | |

### Example 4: pH 6 composition stability

Composition H of example 1 was packaged in either a PET amber vial or a PET white opaque vial and subjected to stability analysis as shown in the tables 6 and 7 below:

**Table 6**

| **Composition H in a PET white opaque vial** | | | | | | | | | | | |
|---|---|---|---|---|---|---|---|---|---|---|---|
| ***Test*** | ***Specificatio n*** | ***Initial time*** | ***1 month 25°C*/*60 % RH*** | ***3 months 25°C*/*60 % RH*** | ***6 months 25°C*/*60 % RH*** | ***1 month 40°C*/*75 % RH*** | ***3 months 40°C*/*75 % RH*** | ***6 months 40°C*/*7 5% RH*** | ***15 days 60°C*** | ***1 month 60°C*** | ***ICH Photostabilit y (primary packaging)*** |
| Appearance of solution | Clear, colourless solution | Complie s | Complie s | Complies | Complies | Complies | Light yellow | Light yellow | Light yellow | Light yellow | Complies |
| pH (as is) | Report result | 6.3 | TNP | 5.7 | 6.2 | TNP | 5.8 | 5.9 | TNP | 5.5 | 6.1 |
| Relative Density at 20°C | Report result | 1.106 | TNP | TNP | 1.110 | TNP | TNP | 1.109 | TNP | 1.106 | 1.109 |
| Assay - HPLC*: Ketoprofen Lysine salt | 90.0 - 110.0% of label claim | 99.7% | 98.6% | 102.0% | 100.9% | 102.2% | 102.1% | 102.0% | 99.8% | 100.4 % | 100.9^% |

| Related Substances (HPLC) - (Reporting threshold ≥ 0.10%) | | | | | | | | | | | |
|---|---|---|---|---|---|---|---|---|---|---|---|
| Any unspecified degradation product | Report result | < 0.10% | < 0.10% | < 0.10% | < 0.10% | < 0.10% | < 0.10% | < 0.10% | < 0.10% | < 0.10% | < 0.10% |
| Total degradation product | Report result | < 0.10% | < 0.10% | < 0.10% | < 0.10% | < 0.10% | < 0.10% | < 0.10% | < 0.10% | < 0.10% | < 0.10% |
| Assay - HPLC: Sodium Methylparahydrox ybenzoate | 90.0 - 110.0% of label claim | 92.1% | 85.8% | 92.0% | 87.6% | 88.9% | 86.0% | 81.0% | 76.0% | 72.4% | 92.0% |

| | | | | | | | | | | | |
|---|---|---|---|---|---|---|---|---|---|---|---|
| *as free acid TNP = Test not performed | | | | | | | | | | | |

**Table 7**

| **Composition H in a PET amber vial** | | | | | | | |
|---|---|---|---|---|---|---|---|
| ***Test*** | ***Specification*** | ***Initial time*** | ***1 month 40°C*/*75% RH*** | ***3 months 40°C*/*75% RH*** | ***15 days 60°C*** | ***1 month 60°C*** | ***ICH Phostability (primary packaging)*** |
| Appearance of solution | Clear, colourless solution | Complies | Complies | Light yellow | Light yellow | Light yellow | White cloudy |
| pH (as is) | Report result | 6.3 | TNP | 5.7 | TNP | 5.8 | 5.7 |
| Relative Density at 20°C | Report result | 1.106 | TNP | 1.108 | TNP | 1.110 | 1.108 |
| Assay - HPLC*: Ketoprofen Lysine salt | 90.0 - 110.0% of label claim | 99.7% | 98.5% | 101.2% | 101.4% | 101.8% | 78.5%** |

| Related Substances (HPLC) - (Reporting threshold ≥ 0.10%) | | | | | | | |
|---|---|---|---|---|---|---|---|
| Any unspecified degradation product | Report result | < 0.10% | < 0.10% | < 0.10% | < 0.10% | < 0.10% | 1.33% (Imp. A) |
| Total degradation product | Report result | < 0.10% | < 0.10% | < 0.10% | < 0.10% | < 0.10% | 1.45% |
| Assay - HPLC: Sodium Methylparahydroxybenzoate | 90.0 - 110.0% of label claim | 92.0% | 88.6% | 86.1% | 78.5% | 76.1% | 88.1% |

| | | | | | | | |
|---|---|---|---|---|---|---|---|
| *as free acid ** due to precipitation of API TNP = Test not performed | | | | | | | |

### Example 5: pH 6.5 composition stability

| **Composition K** | **% w/V (g/L)** |
|---|---|
| Ketoprofen Lysine salt | 0.572 |
| Liquid sorbitol 70% nc | 45.000 |
| Sodium saccharine | 0.070 |
| Licorice flavour 2742/AM | 0.050 |
| Mint flavour 2621/AZ | 0.150 |
| Methyl-p-hydroxybenzoate sodium | 0.115 |
| Sodium phosphate monobasic dihydrate | q.s. pH=6.5 |
| Purified water | q.s. to 100 |

Composition K was packaged in either a PET amber vial or a PET white opaque vial and subjected to stability analysis as shown in the tables 8 and 9 below:

**Table 8**

| **Composition K in a PET white opaque vial** | | | | | | | | | | | |
|---|---|---|---|---|---|---|---|---|---|---|---|
| ***Test*** | ***Specificatio n*** | ***Initial time*** | ***1 month 25°C*/*60 % RH*** | **3 *months 25°C*/*60 % RH*** | **6 *months 25°C*/*60 % RH*** | ***1 month 40°C*/*75 % RH*** | 3 ***months 40°C*/*75 % RH*** | 6 ***months 40°C*/*75 % RH*** | ***15 days 60°C*** | ***1 month 60°C*** | ***ICH Phostability (primary packaging)*** |
| Appearance of solution | Clear, colourless solution | Complies | Complies | Complie s | Complie s | Complie s | Light yellow | Light yellow | Light yellow | Light yellow | Complies |
| pH (as is) | Report result | 6.8 | TNP | 6.3 | 6.6 | TNP | 6.3 | 6.2 | TNP | 5.7 | 6.6 |
| Relative Density at 20°C | Report result | 1.106 | TNP | TNP | 1.109 | TNP | TNP | 1.120 | TNP | 1.109 | 1.108 |
| Assay - HPLC*: Ketoprofen Lysine salt | 90.0-110.0% of label claim | 100.7% | 101.4% | 101.5% | 101.9% | 103.1 % | 103.4% | 102.7% | 100.2 % | 100.5 % | 100.3^% |

| Related Substances (HPLC) - (Reporting threshold ≥ 0.10%) | | | | | | | | | | | |
|---|---|---|---|---|---|---|---|---|---|---|---|
| Any unspecified degradation product | Report result | < 0.10% | < 0.10% | < 0.10% | < 0.10% | < 0.10% | < 0.10% | < 0.10% | < 0.10% | < 0.10% | < 0.10% |
| Total degradation product | Report result | < 0.10% | < 0.10% | < 0.10% | < 0.10% | < 0.10% | < 0.10% | < 0.10% | < 0.10% | < 0.10% | < 0.10% |
| Assay - HPLC: Sodium Methylparahydr oxybenzoate | 90.0-110.0% of label claim | 92.5% | 91.1% | 90.1% | 86.3% | 86.6% | 80.7% | 71.3% | 67.9% | 63.0% | 91.1% |

| | | | | | | | | | | | |
|---|---|---|---|---|---|---|---|---|---|---|---|
| *as free acid TNP = Test not performed | | | | | | | | | | | |

**Table 9**

| **Composition K *in a PET amber vial*** | | | | | | | |
|---|---|---|---|---|---|---|---|
| ***Test*** | ***Specific ation*** | ***Initial time*** | ***1 month 40°C*/*7 5% RH*** | ***3 month* s *40°C*/*7 5% RH*** | ***15 days 60°C*** | ***1 mon th 60°C*** | ***ICH Phostabily y (primary packagin g*)** |
| Appearance of solution | Clear, colourle ss solution | Comp lies | Compl ies | Light yellow | Ligh t yello w | Ligh t yello w | White cloudy |
| pH (as is) | Report result | 6.8 | TNP | 6.3 | TNP | 5.8 | 5.7 |
| Relative Density at 20°C | Report result | 1.109 | TNP | 1.108 | TNP | 1.11 1 | 1.108 |
| Assay - HPLC*: Ketoprofen Lysine salt | 90.0 - 110.0% of label claim | 100.7 % | 98.9% | 101.8 % | 102. 3% | 101. 1% | 75.2%** |

| Related Substances (HPLC) - (Reporting threshold ≥ 0.10%) | | | | | | | |
|---|---|---|---|---|---|---|---|
| Any unspecified degradation product | Report result | < 0.10% | < 0.10% | < 0.10% | < 0.10 % | < 0.10 % | 1.60% (Imp. A) |
| Total degradation product | Report result | < 0.10% | < 0.10% | < 0.10% | < 0.10 % | < 0.10 % | 1.71% |
| Assay - HPLC: Sodium Methylparahydroxy benzoate | 90.0 - 110.0% of label claim | 92.5% | 83.7% | 79.8% | 73.0 % | 66.5 % | 87.7% |

| | | | | | | | |
|---|---|---|---|---|---|---|---|
| *as free acid ** due to precipitation of API TNP = Test not performed | | | | | | | |

## Claims

1. A pharmaceutical composition in the form of an oral aqueous solution comprising ketoprofen lysine salt, sorbitol, sodium saccharin, mint flavour, licorice flavour, methyl-4-hydroxybenzoate sodium, and having a pH comprised between 5.5 and 6.5.

2. The composition of claim 1, wherein the pH is 6.

3. The composition of claims 1-2, wherein the pH has been obtained by addition with monobasic sodium phosphate.

4. The composition of claims 1-3, packaged in a container that protects its content from UV radiations.

5. The composition of claim 4, wherein the container consists of amber glass, amber PET or non-translucent PET.

6. The composition of claims 1-5, wherein w/V percentages expressed in g/L are as follows:
ketoprofen lysine salt 0.572 %
liquid sorbitol (70% solution) 45 %
sodium saccharin 0.07 %
methyl-4-hydroxybenzoate sodium 0.115 %.

7. The composition of claim 6 wherein the total volume of the solution equals 7 mL.

8. The composition of claims 1-7 for use as medicament.

## Patentansprüche

1. Pharmazeutische Zusammensetzung in der Form einer oralen, wässrigen Lösung, die Ketoprofen-Lysinsalz, Sorbitol, Natriumsaccharin, Minzaroma, Lakritzaroma, Natrium-methyl-4-hydroxybenzoat umfasst und einen pH-Wert, der zwischen 5,5 und 6,5 umfasst ist, aufweist.

2. Zusammensetzung nach Anspruch 1, wobei der pH-Wert 6 ist.

3. Zusammensetzung nach den Ansprüchen 1-2, wobei der pH-Wert durch Zugabe von monobasischem Natriumphosphat erhalten wurde.

4. Zusammensetzung nach den Ansprüchen 1-3, die in einem Behälter gepackt ist, der seinen Inhalt vor UV-Strahlen schützt.

5. Zusammensetzung nach Anspruch 4, wobei der Behälter aus bernsteinfarbenem Glas, bernsteinfarbenem PET oder nicht-durchscheinendem PET besteht.

6. Zusammensetzung nach den Ansprüchen 1-5, wobei die Gew./Vol.-Prozentanteile, ausgedrückt in g/l, wie folgt sind:
Ketoprofen-Lysinsalz 0,572%
flüssiges Sorbitol (70%ige Lösung) 45%
Natriumsaccharin 0,07%
Natriummethyl-4-hydroxybenzoat 0,115%.

7. Zusammensetzung nach Anspruch 6, wobei das Gesamtvolumen der Lösung gleich zu 7 mL ist.

8. Zusammensetzung nach den Ansprüchen 1-7 zur Verwendung als Medikament.

## Revendications

1. Composition pharmaceutique sous la forme d'une solution aqueuse orale comprenant un sel de lysine de kétoprofène, du sorbitol, de la saccharine sodique, de l'arôme de menthe, de l'arôme de réglisse, du 4-hydroxybenzoate de méthyle sodique, et ayant un pH compris entre 5,5 et 6,5.

2. Composition selon la revendication 1, dans laquelle le pH est de 6.

3. Composition selon les revendications 1-2, dans laquelle le pH a été obtenu par ajout de dihydrogénophosphate de sodium.

4. Composition selon les revendications 1-3, conditionnée dans un récipient qui protège son contenu des radiations UV.

5. Composition selon la revendication 4, dans laquelle le récipient consiste en verre ambré, en PET ambré ou en PET non translucide.

6. Composition selon les revendications 1-5, dans laquelle les pourcentages en p/V exprimés en g/L sont tels que ce suit :
sel de lysine de kétoprofène 0,572 %
sorbitol liquide (solution à 70 %) 45 %
saccharine sodique 0,07 %
4-hydroxybenzoate de méthyle sodique 0,115 %

7. Composition selon la revendication 6 dans laquelle le volume total de la solution est égal à 7 mL.

8. Composition selon les revendications 1-7 destinée pour l'utilisation comme médicament.
